(19) Europäisches Patentamt
European Patent Office
Office européen des brevets

(11) EP 0 694 520 B1

(12) **FASCICULE DE BREVET EUROPEEN**

(45) Date de publication et mention de la délivrance du brevet:
**06.10.1999 Bulletin 1999/40**

(51) Int. Cl.$^6$: **C07C 47/225**, C07C 255/31, C07C 33/12, C07C 43/162, C07C 251/40, C11B 9/00

(21) Numéro de dépôt: **95111396.8**

(22) Date de dépôt: **20.07.1995**

(54) **Dérivés de l'aldéhyde campholénique et leur utilisation en parfumerie**

Campholen-Aldehyd-Derivate und ihre Anwendung in der Parfümerie

Campholenic aldehyde derivative and their use in perfumery

(84) Etats contractants désignés:
**CH DE ES FR GB IT LI NL**

(30) Priorité: **28.07.1994 CH 238594**

(43) Date de publication de la demande:
**31.01.1996 Bulletin 1996/05**

(73) Titulaire: **FIRMENICH SA**
**1211 Genève 8 (CH)**

(72) Inventeurs:
- **Chapuis, Christian**
  **CH-1295 Mies (CH)**
- **Blanc, Pierre-Alain**
  **CH-1263 Crassier (CH)**

(74) Mandataire:
**Salvaterra-Garcia, Maria de Lurdes et al**
**Firmenich SA**
**Département des Brevets**
**Case Postale 239**
**1211 Genève 8 (CH)**

(56) Documents cités:
**EP-A- 0 155 591**
**WO-A-93/21142**
**DE-A- 4 131 119**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 694 520 B1

## Description

La présente invention a trait au domaine de la parfumerie et plus particulièrement à celui des composés odorants dérivés de l'aldéhyde campholénique.

L'art antérieur fait état d'un grand nombre de composés de ce type, en particulier d'alcools caractérisés par des odeurs de type santalé. Ceci est une conséquence du fait que, malgré l'abondance de tels composés connus à ce jour, et la contribution que chacun peut apporter à la reconstitution olfactive des caractères de l'huile naturelle de santal, l'odeur typique de cette dernière ne peut pas être remplacée par l'un quelconque de ces composés, ni même par un mélange de plusieurs. Ce qui explique que la recherche dans ce domaine reste soutenue et que de nouveaux produits viennent s'ajouter régulièrement à la palette du parfumeur dans ce domaine.

Ainsi, dans une publication récente, à savoir la demande de brevet international n° WO 93/21142, on fait état de composés de formule

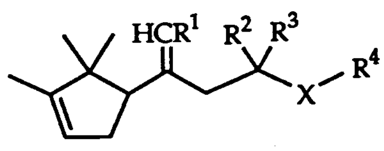

dans laquelle, indépendamment les uns des autres, $R^1$ représente l'hydrogène ou un radical méthyle, $R^2$ et $R^3$ sont hydrogène ou un radical alkyle ayant de 1 à 5 atomes de carbone, $R^4$ est l'hydrogène ou un groupe $CHR^5R^6$, dans lequel $R^5$ et $R^6$ représentent l'hydrogène ou un radical alkyle ayant de 1 à 6 atomes de carbone, et X représente un groupe CO ou CHOH, étant entendu que a) au moins l'un des symboles $R^2$ et $R^3$ représente un radical alkyle et b) le 2-méthyl-4-(2,2,3-triméthyl-3-cyclopentén-1-yl)-4-pentén-1-ol est exclu.

Il est, par ailleurs, indiqué dans ce document que les composés dans lesquels $R^2$ et $R^3$ représentent les deux des groupes alkyles sont préférés à leurs homologues dans lesquels au moins l'un de ces groupes représente l'hydrogène, leur note santalée étant plus puissante.

La formule décrite dans ce document de l'art antérieur possède un caractère extrêmement général; en effet, elle peut être interprétée comme englobant potentiellement des centaines de composés et, notamment, le 2-méthyl-4-(2',2',3'-triméthyl-3'-cyclopentén-1'-yl)-4-pentén-1-al, l'un des composés qui font l'objet de la présente description. En fait, il ressort très clairement du document susmentionné que ses auteurs n'ont pas du tout reconnu la valeur potentielle de ce composé. Non seulement il n'est point décrit dans la demande citée, mais, en plus, il apparaît à l'évidence que, conformément à la préférence indiquée pour les composés doublement substitués dans la chaîne latérale, ces auteurs se sont concentrés sur la préparation de ces derniers, et en particulier de leurs variantes à fonction hydroxylée ou cétonique, comme il ressort des exemples spécifiques décrits dans ledit document. Par ailleurs, on ne peut pas y trouver, à part l'indication de préférence mentionnée ci-dessus, la moindre suggestion qu'un composé en particulier, parmi tous ceux que la formule peut inclure, pourrait se comporter olfactivement de façon tout à fait distincte de celle des composés décrits dans l'art antérieur. Rien dans ce document ne laissait ainsi soupçonner le résultat que nous décrivons à présent, c'est-à-dire que le 2-méthyl-4-(2',2',3'-triméthyl-3'-cyclopentén-1'-yl)-4-pentén-1-al possède des qualités olfactives tout à fait surprenantes et atypiques pour ce type de structure, au vu de l'art antérieur. Or, c'est exactement ce que nous venons de constater.

L'invention a pour objet un composé de formule

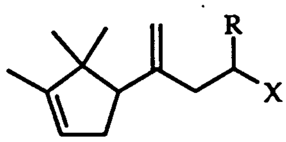

(I)

dans laquelle R représente l'hydrogène ou un radical méthyle et X représente un groupe -CHO ou - C≡N.

Parmi les composés (I), nous avons en effet découvert que le 2-méthyl-4-(2',2',3'-triméthyl-3'-cyclopentén-1'-yl)-4-pentén-1-al, et plus particulièrement son isomère chiral de configuration (-)-(1'R), qui est préféré selon l'invention, possède une odeur surprenante vis-à-vis des connaissances de l'art antérieur. Ce composé développe une note boiséesantalée, légèrement aldéhydée, rappelant plus la connotation cèdre-pin que la connotation santal, note qui se trouve combinée avec un net caractère marin et ozoné. Ce dernier se trouve encore renforcé dans la note de fond, qui est fortement ozonée-marine, rappelant l'odeur des algues. Cette combinaison de caractères était tout à fait inattendue

au vu des connaissances de l'art pour ce type de composés. Ses qualités olfactives sont d'autant plus importantes qu'elles permettent à ce composé d'apporter aux notes santalées un effet marin et aqueux très prisé, particulièrement puissant et frais en tête. Il impartit ainsi aux compositions l'effet dit "transparent" des notes marines, très apprécié actuellement du public et connu dans des parfums à connotation verte ou florale, mais pas du tout dans les produits fragrants à caractère boisé-santal. Par ailleurs, sa note odorante est dotée d'une bonne puissance et substantivité.

La présence de ces caractères marin, ozoné, algues dans l'odeur de ce type de composés semble d'ailleurs être très rare puisque, malgré le nombre de composés homologues et analogues préparés pendant les recherches qui ont abouti à ce résultat inattendu, seul un homologue inférieur du composé susmentionné, à savoir le 4-(2',2',3'-triméthyl-3'-cyclopentén-1'-yl)-4-pentén-1-al, possède une odeur à caractère légèrement aqueux, quoique, curieusement, non plus combiné à des notes santalées, mais à des caractères odorants complètement distincts, caractères qui, de surcroît, ne sont pas les mêmes chez les deux isomères chiraux de ce composé. C'est ainsi que le (-)-(1'R)-4-(2',2',3'-triméthyl-3'-cyclopentén-1'-yl)-4-pentén-1-al développe une odeur où l'on trouve une note aldéhydée, rappelant celle du citronellal, hespéridée, épicée, ressemblant à l'odeur de la feuille de citronnelle. Sa note de fond possède un caractère rappelant l'odeur du 3-(4-tert-butyl-1-phényl)-2-méthylpropanal, mais avec une connotation encore plus verte, et un léger côté orangé. Son énantiomère, (+)-(1'S)-4-(2',2',3'-triméthyl-3'-cyclopentén-1'-yl)-4-pentén-1-al, possède lui une odeur plus métallique et aldéhydée, rappelant l'odeur du trans-4-décénal, sa note de fond étant plus fleurie et plus intense dans le caractère aqueux. Son utilisation pour renforcer ce dernier dans des compositions et articles parfumés est d'ailleurs préférée à celle de son énantiomère.

D'autre part, les nitriles de formule (I) ne possèdent plus cet aspect marin dans leur odeur. Par exemple, le 2-méthyl-4-(2',2',3'-triméthyl-3'-cyclopentén-1'-yl)-4-pentènenitrile et, en particulier, son isomère de configuration (-)-(1'R), qui est préféré, possède une odeur boisée-hespéridée, fruitée et un peu grasse. L'aspect hespéridé, fruité, frais se trouve encore renforcé dans l'odeur du (-)-(1'R,2R)-2-méthyl-4-(2',2',3'-triméthyl-3'-cyclopentén-1'-yl)-4-pentènenitrile, et accompagné d'un caractère de fond santalé. Ce composé est d'ailleurs préféré, d'un point de vue olfactif, à son diastéréomère de configuration (-)-(1'R,2S), dont l'odeur santalée ne possède plus le caractère hespéridé et fruité désiré.

Les composés de l'invention sont l'exemple même de l'imprévisibilité du comportement olfactif sur la base de la similarité de structure car, non seulement leurs odeurs sont distinctes de celles des composés connus, mais encore tout à fait différentes d'un composé à l'autre, et même entre isomères chiraux d'un même composé.

De par leurs qualités olfactives, les composés de l'invention se révèlent très utiles pour la préparation de compositions parfumantes et articles parfumés qui font également l'objet de l'invention. Ils sont aussi avantageux pour des applications en parfumerie fine qu'en parfumerie fonctionnelle, car ils se prêtent au parfumage d'articles aussi divers que les parfums et eaux de toilette, les savons, les gels de douche ou bain, les shampoings ou autres articles destinés au traitement et à l'hygiène des cheveux, les préparations cosmétiques, les désodorisants corporels ou d'air ambiant, les détergents ou adoucissants textiles ou, encore, les produits d'entretien. Dans ces applications, ils peuvent être employés soit seuls, soit, comme il est plus courant en parfumerie, en mélange avec d'autres ingrédients parfumants, des solvants ou des adjuvants d'usage courant en parfumerie et que l'homme du métier est à même de choisir en fonction de l'effet désiré et de la nature du produit à parfumer. Une description détaillée de tels ingrédients est ici superflue et on peut citer à titre d'exemple des ingrédients tels que ceux décrits dans des ouvrages de référence comme celui de S. Arctander, Perfume and Flavor Chemicals, Montclair, N.J. USA (1969).

Les concentrations dans lesquelles les composés de l'invention peuvent être utilisés pour obtenir les effets parfumants désirés varient dans une gamme de valeurs très étendue, étant bien connu que ces valeurs dépendent et de la nature de l'article à parfumer et de l'effet odorant recherché, ainsi que de la nature des autres coingrédients parfumants dans une composition donnée. C'est ainsi que des concentrations de l'ordre de 1 à 5%, voire 10 ou même 20% en poids dudit composé de l'invention, par rapport au poids de la composition, sont tout à fait appropriées lorsque les composés cités sont ajoutés à des compositions parfumantes variées. Des concentrations nettement inférieures à ces valeurs peuvent être employées lorsqu'ils sont utilisés pour le parfumage d'articles divers tels que ceux cités plus haut.

Les composés (I) selon l'invention sont préparés par un procédé original faisant l'objet de l'invention, caractérisé en ce qu'on soumet un éther de formule

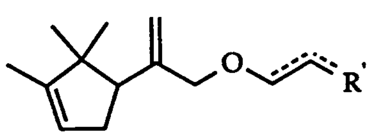

(II)

possédant une double liaison dans l'une des positions indiquées par les lignes pointillées et dans laquelle R' représente l'hydrogène, un radical méthyle ou un radical méthylène, à un traitement thermique pour former un aldéhyde de formule

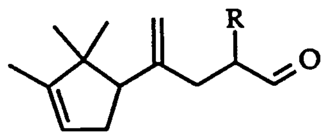

(I')

dans laquelle R représente l'hydrogène ou un radical méthyle et, le cas échéant, on transforme ledit aldéhyde en le nitrile correspondant, de façon connue en soi.

Le traitement thermique qui caractérise le procédé de l'invention est une réaction du type réarrangement de Claisen qui a lieu à des températures comprises entre environ 160° et 240°C. Les éthers de départ (II) sont des composés nouveaux qui peuvent être préparés par éthérification du 2-(2',2',3-triméthyl-3'-cyclopentén-1'-yl)-2-propén-1-ol dans les conditions décrites en détail plus loin.

Selon un mode d'exécution particulier du procédé de l'invention, avantageux pour la préparation du 2-méthyl-4-(2',2',3'-triméthyl-3'-cyclopentén-1'-yl)-4-pentén-1-al, on soumet le 4-[1-(allyloxyméthyl)éthényl]-1,5,5-triméthyl-1-cyclopentène à un traitement thermique, en présence d'un catalyseur, pour obtenir ledit 2-méthyl-4-(2',2',3'-triméthyl-3'-cyclopentén-1' - yl)-4-pentén-1-al.

En tant que catalyseur, on peut utiliser par exemple du Pd/C ou du Rh/C, ou encore le dichlorure de tris(triphénylphosphine)ruthénium. La réaction s'effectue à une température variant dans une gamme de valeurs assez large, par exemple de 130° à 250°C. De même, le catalyseur peut être employé dans une gamme de concentrations variant entre 0,05 et 0,5 mole%, par rapport à l'éther de départ. De meilleurs rendements ont été obtenus lorsqu'on a utilisé le catalyseur à une concentration comprise entre 0,05 et 0,2 mole%. Selon la nature du catalyseur utilisé, la réaction peut être conduite en l'absence de solvant, comme c'est le cas avec le dichlorure de tris(triphénylphosphine) ruthénium, ou en présence d'un milieu de dilution approprié, choisi parmi les solvants d'usage courant pour ce type de réaction. Par exemple, lors de l'utilisation de Pd/C ou Ru/C, nous avons trouvé qu'il était avantageux d'utiliser le mesitylène comme solvant. On peut également employer le toluène sous pression.

Cette voie avantageuse du procédé de l'invention permet de synthétiser, en un pot, le penténal susmentionné, à partir de l'éther (II) allylique cité, dont la préparation est plus aisée et économique que celle de son isomère 1,5,5-triméthyl-4-[1-(1-propényloxyméthyl)éthényl]-1-cyclopentène, possédant la double liaison en position intermédiaire. Le 4-[1-(allyloxyméthyl)éthényl]-1,5,5-triméthyl-1-cyclopentène, un composé nouveau, est en effet préparé par allylation, à l'aide de bromure d'allyle, du 2-(2',2',3'-triméthyl-3'-cyclopentén-1'-yl)-2-propén-1-ol mentionné plus haut, dans des conditions décrites en détail plus loin. Lorsque cet éther est chauffé en présence du catalyseur, il se transforme d'abord en son isomère 1,5,5-triméthyl-4-[1-(1-propényloxyinéthyl)éthényl]-1-cyclopentène mentionné ci-dessus, lequel subit ensuite le réarrangement de Claisen pour former l'aldéhyde désiré.

Il va de soi que l'utilisation du propénol de départ sous forme de ses isomères chiraux (-)-(S) et (+)-(R), lesquels peuvent être obtenus à partir des isomères optiquement actifs correspondants de l'aldéhyde campholénique, selon la méthode décrite dans EP 155 591, permet d'obtenir les isomères chiraux correspondants des éthers (II), et par la suite des aldéhydes (I'), via le procédé de l'invention.

Les aldéhydes (I') peuvent ensuite être utilisés comme produits de départ pour la préparation des nitriles de formule (I) selon l'invention. Cette transformation peut être obtenue à l'aide de réactions de type classique, typiquement par l'intermédiaire de l'oxime de l'aldéhyde de départ que l'on fait réagir ensuite avec un agent déshydratant, par exemple l'anhydride acétique. La réaction peut être réalisée en un pot, dans des conditions connues et décrites plus loin ou, alternativement, on peut d'abord isoler l'oxime, qui est un composé nouveau faisant également l'objet de l'invention, et la soumettre ensuite à l'action de l'agent déshydratant. D'autres agents déshydratants que celui cité plus haut peuvent bien entendu être employés. Une énumération de ces réactifs est ici superflue, ce type de réactions étant bien connu de l'homme de métier [voir, par exemple, J. March, Advanced Organic Chemistry : Reactions, Mechanisms and Structure, 3rd ed., sections 6-21 et 7-40, John Wiley & Sons, USA (1985)].

Les aldéhydes (I') sont aussi utiles pour la préparation des alcools odorants correspondants. Puisque le procédé de l'invention permet l'obtention de ces aldéhydes dans une forme optiquement pure, il est aussi possible d'obtenir les isomères chiraux purs desdits alcools. Ainsi, l'invention a aussi pour objet l'utilisation d'un aldéhyde de formule (I') telle que définie auparavant à titre de produit de départ pour la préparation de l'alcool correspondant, caractérisée en ce qu'on traite ledit aldéhyde avec un agent réducteur. Selon un mode d'exécution particulier, on utilise le 2-méthyl-4-(2',2',3'-triméthyl-3'-cyclopentén-1'-yl)-4-pentén-1-al pour la préparation du 2-méthyl-4-(2',2',3'-triméthyl-3'-cyclopentén-1'-yl)-4-pentén-1-ol.

Le composé (I') susmentionné ouvre en effet une voie nouvelle et avantageuse pour la préparation de cet alcool odorant décrit dans EP 155 591. Selon cette référence, on préparait cet alcool comme il est décrit au Schéma I ci-après :

SCHEMA I

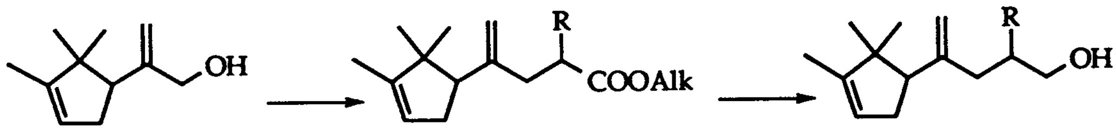

Alk = alkyle

$R = H, CH_3$

Alk=alkyle
$R = H, CH_3$

Or, malgré l'utilité et les mérites du procédé connu, nous avons découvert que l'utilisation du 2-méthyl-4-(2',2',3'-triméthyl-3'-cyclopentén-1'-yl)-4-pentén-1-al selon l'invention, à la place du 2-méthyl-4-(2',2',3'-triméthyl-3'-cyclopentén-1'-yl)-4-penténoate d'éthyle, l'un des esters intermédiaires dans ce schéma, permettait d'améliorer la synthèse de l'alcool, car il présentait plusieurs avantages sur le procédé connu. Ceci résulte du fait que ce nouveau procédé permet non seulement d'éviter l'excès d'hydrure dans la phase finale de préparation de l'alcool susmentionné, qui résultait de la réduction de l'ester cité à l'aide de $LiAlH_4$, mais d'éliminer également l'utilisation de l'orthopropanoate d'éthyle, un composé fort onéreux et nécessaire à la préparation dudit ester ou 2-méthyl-4-(2',2',3'-triméthyl-3'-cyclopentén-1'-yl)-4-penténoate d'éthyle.

Par ailleurs, selon le procédé de l'invention, et contrairement à celui de l'art antérieur, en tant qu'agent réducteur on peut utiliser un composé quelconque d'usage courant dans la transformation d'aldéhydes en alcools, ce qui permet de choisir des agents réducteurs moins onéreux que le $LiAlH_4$.

Bien entendu, l'utilisation du 2-méthyl-4-(2',2',3'-triméthyl-3'-cyclopentén-1'-yl)-4-pentén-1-al sous forme de l'un de ses énantiomères de configuration (-)-(1'R) et (+)-(1'S) permet d'obtenir les isomères optiquement actifs correspondants du 2-méthyl-4-(2',2',3'-triméthyl-3'-cyclopentén-1'-yl)-4-pentén-1-ol.

Le composé susmentionné a été décrit dans le brevet EP 155 591, où l'on indique qu'il possède une odeur s'apparentant à celle de l'essence de santal naturelle. Par ailleurs, un isomère optiquement actif de ce composé, à savoir le (-)-2-méthyl-4-(2',2',3'-triméthyl-3'-cyclopentén-1'-yl)-4-pentén-1-ol, est également divulgué, mais aucune description de ses propriétés olfactives propres ne peut être trouvée dans cette référence. En fait, malgré la valeur olfactive de chacun des isomères, nous avons constaté que leurs propriétés odorantes diffèrent d'un isomère à l'autre et nous avons maintenant découvert que l'isomère susmentionné possède une odeur plus proche de celle de l'essence de santal naturelle que son énantiomère ayant une rotation optique positive.

Par ailleurs, chacun de ces composés est un mélange de deux diastéréomères, de par les deux orientations possibles du radical méthyle substituant dans la chaîne latérale. Or, il s'est avéré que parmi les quatre diastéréomères du 2-méthyl-4-(2',2',3'-triméthyl-3'-cyclopentén-1'-yl)-4-pentén-1-ol, qui sont des composés nouveaux, le (-)-(1'R,2R)-2-méthyl-4-(2',2',3'-triméthyl-3'-cyclopentén-1'-yl)-4-pentén-1-ol

ayant la formule

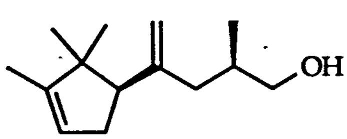

(-)-(1'R,2R)

est préféré des' parfumeurs pour sa note plus santalée et élégante et aussi plus puissante. Son diastéréomère de configuration (-)-(1'R,2S) de formule

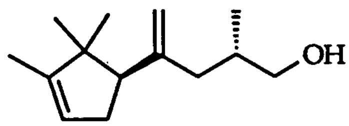

(-)-(1'R,2S)

possède lui une note moins santalée, accompagnée d'un caractère boisé-cèdre plus marqué. Ces deux diastéréomères sont, par ailleurs, plus prisés olfactivement que leurs énantiomères respectifs, à savoir les composés de configuration (+)-(1'S,2S) et (+)-(1'S,2R) de formules

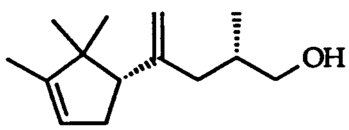

(-)-(1'S,2S)

et

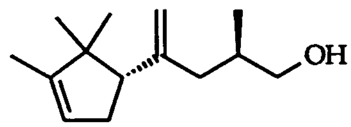

(-)-(1'S,2R)

dont les odeurs sont moins riches en caractère de type lait de santal. Ceci expliquerait le fait mentionné plus haut, c'est-à-dire que les mélanges des deux diastéréomères cités en premier lieu, constituant le (-)-(1'R)-2-méthyl-4-(2',2',3'-triméthyl-3'-cyclopentén-1'-yl)-4-pentén-1-ol de formule

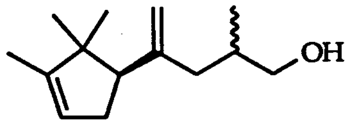

trouvent également plus de faveur auprès des parfumeurs que les mélanges des deux autres diastéréomères formant le (+)-(1'S)-2-méthyl-4-(2',2',3'-triméthyl-3'-cyclopentén-1'-yl)-4-pentén-1-ol de formule

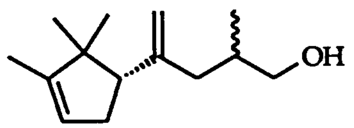

.

Les quatre composés nouveaux susmentionnés, dont les qualités odorantes spécifiques étaient insoupçonnées jusqu'à maintenant, permettent d'obtenir des compositions parfumantes dont la performance olfactive peut être améliorée par rapport à celle des compositions décrites dans l'art antérieur, par exemple en dosant à volonté la quantité de l'isomère préféré de configuration (-)-(1'R,2R) présent dans les mélanges de deux ou plusieurs diastéréomères utilisés en tant qu'ingrédients parfumants dans lesdites compositions, ou en ajoutant cet isomère directement à ces dernières.

Dans ce contexte, il convient de noter que l'utilisation du (-)-(1'R)-2-méthyl-4-(2',2',3'-triméthyl-3'-cyclopentén-1'-yl)-4-pentén-1-al selon l'invention pour préparer l'alcool correspondant, ou (-)-(1'R)-2-méthyl-4-(2',2',3'-triméthyl-3'-cyclopentén-1'-yl)-4-pentén-1-ol, présente un avantage supplémentaire sur celle du procédé connu décrit dans EP 155 591 et illustré au Schéma I. On a en effet constaté que le penténal optiquement actif susmentionné pouvait être plus facilement épimérisé que l'ester intermédiaire du procédé connu, pour être ainsi enrichi en le diastéréomère de configuration 2R correspondant au diastéréomère préféré de l'alcool. L'aldéhyde décrit à présent a donc l'utilité supplémentaire de permettre la préparation à l'échelle industrielle d'une qualité améliorée de cet alcool odorant, et ceci par le biais d'un procédé qui fait usage de réactifs moins complexes et onéreux.

L'invention sera maintenant décrite plus en détail à l'aide des exemples suivants dans lesquels les températures sont indiquées en degrés centigrades et les abréviations ont le sens usuel dans l'art.

Exemple 1

Préparation de (-)-(1'R)-2-méthyl-4-(2',2',3'-triméthyl-3'-cyclopentén-1'-yl)-4-pentén-1-al

A. En un pot à partir de (-)-(S)-4-[1-(allyloxyméthyl)éthényl]-1,5,5-triméthyl-1-cyclopentène

On a chauffé à 180° l'allyl éther susmentionné (1,0 g, 5 mmole) et le dichlorure de tris(triphénylphosphine)ruthénium (6 mg, 0,12 mole%). Après 75 min, on a distillé le mélange réactionnel au four à boules pour obtenir le (-)-(1'R)-2-méthyl-4-(2',2',3'-triméthyl-3'-cyclopentén-1'-yl)-4-pentén-1-al désiré à l'état pur (mélange de deux diastéréomères 2S/2R 55:45; rend. 69%).

P. éb. : 51°/9 Pa
$[\alpha]^{20}_D$ (pur) = -72,0°
IR : 2960, 2710, 1730, 1640, 1460, 1360, 1120, 1015, 900 $cm^{-1}$
RMN($^1H$,360MHz,$CDCl_3$) :

(1'R,2R) : 0,77(s, 3H) ; 1,08(d, J=7, 3H) ; 1,10(s, 3H) ; 1,60(m, 3H) ; 2,04 (m, 1H) ; 2,20(m, 1H) ; 2,30(m, 1H) ; 2,5(m, 2H) ; 2,6(m, 1H) ; 4,91(s, 1H) ; 4,97(s, 1H) ; 5,28(s, 1H) ; 9,66(d, J=2, 1H) δ ppm
(1'R,2S) : 0,76(s, 3H) ; 1,09(s, 3H) ; 1,11(d, J=7,3H) ; 1,60(m, 3H) ; 2,04(m, 1H) ; 2,20(m, 1H) ; 2,30(m, 1H) ; 2,5(m, 2H) ; 2,6(m, 1H) ; 4,90(s, 1H) ; 4,96(s, 1H) ; 5,28(s, 1H) ; 9,62(d,J=2, 1H) δ ppm

RMN($^{13}C$) :

(1'R,2R) : 12,8(q) ; 13,4(q) ; 21,0(q) ; 27,1(q) ; 34,2(t) ; 38,1(t) ; 44,7(d) ; 47,9(s) ; 55,5(d) ; 113,0(t) ; 121,5(d) ; 146,4(s) ; 147,3(s) ; 204,7(d) δ ppm
(1'R,2S) : 12,8(q) ; 14,0(q) ; 21,0(q) ; 27,0(q) ; 34,1(t) ; 38,1(t) ; 44,9(d) ; 48,0(s) ; 56,3(d) ; 112,7(t) ; 121,5(d) ; 146,6(s) ; 147,3(s) ; 204,8(d) δ ppm

SM :

(1'R,2R) : 206(M+,7), 191(12), 173(21), 149(22), 133(59), 105(100), 91(98), 79(88), 67(43), 55(42), 41(38)
(1'R,2S) : 206(M+,8), 191(10), 73(18), 148(20), 133(59), 107(88), 91(100), 79(90), 67(51), 55(48), 41(41)

Odeur : voir plus haut.

L'aldéhyde ainsi obtenu a été enrichi en diastéréomère de configuration (2R) par épimérisation à l'aide de tert-butoxyde de potassium (3 g, 27 mmole), dans le tert-butanol (15 ml), à 20-25°. Après 5 min, on avait obtenu un produit où le rapport 2R/2S était de 60 : 40.

On a également obtenu l'aldéhyde désiré en chauffant à reflux, dans un ballon sous atmosphère d'azote et avec un séparateur d'eau, pendant 3 h, un mélange contenant l'éther de départ susmentionné (50 g), du mésithylène (150 g) et du ruthénium 5% sur charbon humide (1,5 g ; origine : Degussa). Après filtration du catalyseur et concentration (100°/2x$10^2$ Pa), on a obtenu l'aldéhyde désiré.

L'allyl éther de départ a été préparé ainsi.
On a chauffé à reflux, pendant 2h, un mélange de (-)-(1'S)-2-(2',2',3'-triméthyl-3'-cyclopentén-1'-yl)-2-propén-1-ol ($[a]^{20}_D$ (pur) = - 63,9°, 1,0 g, 6 mmole), bromure d'allyle (0,8 ml, 9 mmole) et NaOH (0,4g, 9 mmole). On a extrait à l'éther et à l'eau jusqu'à neutralité, séché sur $Na_2SO_4$, concentré et distillé au four à boules pour obtenir le (-)-(S)-4-[1-(allyloxyméthyl)éthényl]-1,5,5-triméthyl-1-cyclopentène (pureté : 99% ; rend. 83%), dont les caractères analytiques étaient les suivants :

P. éb.: 57°/0,6x$10^2$ Pa
$[\alpha]^{20}_D$(pur)=-60,55°
IR : 3040, 2960, 1645, 1450, 1360, 1090, 1020, 920 $cm^{-1}$
RMN($^1H$,360MHz,$CDCl_3$) : 0,77(s, 3H); 1,08(s, 3H); 1,60(q, J=2, 3H) ; 2,2-2,4(m, 2H); 2,60(t, J=7, 1H); 3,95(s, 2H); 3,99(m, 2H); 5,02(s, 2H); 5,18(m, 1H); 5,22(m, 1H); 5,30(m, 1H); 5,93(m, 1H) δ ppm
RMN($^{13}C$) : 12,8(q); 21,0(q); 26,8(q); 33,9(t); 47,7(s); 53,5(d); 71,1(t); 73,6(t); 112,5(t); 116,7(t); 121,6(d); 135,0(d); 146,2(s); 147,4(s) δ ppm

SM : 206($M^+$,1), 133(90), 119(27), 105(100), 91(72), 79(53), 67(26), 57(27), 41(26)
Odeur : cardamon, épicée, boisée, citron.

B. A partir de (-)-(S)-1,5,5-triméthyl-4-[1-(1-propényloxyméthyl)éthényl]-1-cyclopentène

L'éther susmentionné a été chauffé à 180° pendant 90 min. Après le traitement usuel, on a obtenu l'aldéhyde désiré avec des caractéristiques identiques à celles décrites sous A.

L'éther de départ a été préparé ainsi.
Un mélange de (-)-(1'S)-2-(2',2',3'-triméthyl-3'-cyclopentén-1'-yl)-2-propén-1-ol (4,15 g, 25 mmole) et $(CH_3COO)_2Hg$ (166 mg, 0,52 mmole) en solution dans du propényléthyléther (12 g, 139 mmole) a été porté à reflux durant 48 h. L'excès de propényléthyléther et éthanol a été distillé. Le résidu a été distillé au four à boules en présence de $K_2CO_3$ (332 mg) sous vide poussé, pour donner 0,35 g de (-)-(S)-1,5,5-triméthyl-4-[1-(propényloxyméthyl)éthényl]-1-cyclopentène (rend. 7%) comme un mélange E/Z 1 : 4.

P. éb.: 75°/1,3x$10^2$ Pa
$[\alpha]^{20}_D$ (pur)= -46,4°
IR : 3040, 2980, 2940, 2880, 1660, 1450, 1180, 1140, 910, 800 $cm^{-1}$
RMN($^1$H,360MHz,$CDCl_3$) :

**Z** : 0,69(s, 3H); 1,09(s, 3H); 1,60(d, J=2, 3H); 2,3(m, 2H); 2,58(t, J=7, 1H); 4,21(s, 2H); 4,4(quint, J=7, 1H) ; 5,03(m, 1H); 5,21(m, 1H); 5,28(s, 1H); 5,93(qd, J=2, 7, 1H) δ ppm
**E** : 0,68(s,3H); 1,07(s, 3H); 1,62(d, J=2, 3H); 2,3(m, 2H); 2,60(t, J=7, 1H); 4,11(s, 2H); 4,8(quint, J=7, 1H); 5,03(m, 1H); 5,21 (m, 1H); 5,28(s, 1H); 6,21(qd, J=2, 11, 1H) δ ppm

RMN($^{13}$C) : δ ppm

**Z** : 9,3(q); 12,8(q); 20,9(q); 26,7(q); 33,8(t); 47,7(s); 53,4(d); 75,0(t); 101(d); 112,8(t); 121,6(d); 145.4(d); 147,4(s) δ ppm
**E** : 12,6(q); 12,8(q); 20,9(q); 26,7(q); 33,8(t); 47,7(s); 53,6(d); 72,6(t); 99,2(d); 113,1(t); 121,6(d); 145,8(s); 146,3(d) δ ppm

SM :

**Z** : 206($M^+$,8), 191(7), 149(16), 133(20), 121(25), 107(92), 93(100), 79(50), 41(29)
**E** : 206($M^+$,8), 191(10), 173(8), 148(16), 133(30), 121(25), 107(86), 93(100), 79(50), 41(29)

Exemple 2

Préparation de (+)-(1'S)-2-méthyl-4-(2',2',3'-triméthyl-3'-cyclopentén-1'-yl)-4-pentén-1-al

Cet aldéhyde a été préparé de façon similaire à celle décrite dans l'Exemple 1, mais en partant du (+)-(1'R)-2-(2',2',3'-triméthyl-3'-cyclopentén-1'-yl)-2-propén-1-ol ($[\alpha]^{20}_D$ (pur) = + 75,2°). On a ainsi obtenu un produit (mélange de diastéréomères 2S/2R 52 : 48) dont les caractères analytiques étaient identiques à ceux de l'aldéhyde décrit dans l'Exemple 1, à l'exception de :

P. éb.: 41°/3,2 Pa
$[\alpha]^{20}_D$ (pur) = + 82,7°
Odeur : boisée, santalée, aldéhydée, légèrement aqueuse.

Exemple 3

Préparation de (+)-(1'S)-4-(2',2',3'-triméthyl-3'-cyclopentén-1'-yl)-4-pentén-1-al

A. A partir de (+)-(1'S)-4-(2',2',3'-triméthyl-3'-cyclopentén-1'-yl)-4-pentén-1-ol

Dans un ballon, on a chargé de la Célite ® (52,5 g), du chlorochromate de pyridinium (PCC, 35 g, 0,162 mole) dans 220 ml de dichlorométhane. On a ensuite introduit, sous $N_2$ et agitation mécanique, 21 g (0,108 mole) de (+)-(1'S)-4-(2',2',3'-triméthyl-3'-cyclopentén-1'-yl)-4-pentén-1-ol en solution dans 20 ml de $CH_2Cl_2$. Après 1 h, on a filtré sur

$SiO_2$ avec éther éthylique, concentré, lavé avec HCl à 15% et ensuite à neutralité avec de l'eau pour obtenir, après évaporation et distillation au four à boules, 19,75 g (pureté > 99%; rend. 95%) du penténal susmentionné, ayant les caractères analytiques suivants :

P. éb.: 60°/33 Pa
$[\alpha]^{20}_D$(pur)= + 68,6°
IR : 2960, 1730, 1440, 900 $cm^{-1}$
RMN($^1H$,360MHz,$CDCl_3$) : 0,75(s, 3H); 1,08(s, 3H); 1,59(q, J=2,1H); 2,2-2,65(m, 7H); 4,83(d, J=2, 1H); 4,91(s, 1H); 5,27(s large, 1H) ; 9,79(t, J=2, 1H) δ ppm
RMN($^{13}C$) : 12,8(q); 20,9(q); 27,0(q); 28,6(t); 33,9(t); 42,4(t); 48,0(s); 56,6(d); 111,2(t); 121,5(d); 147,3(s); 148,1(s); 202,3(d) δ ppm
SM : 192($M^+$,36), 177(28), 159(46), 133(80), 105(99), 91(100), 79(64), 55(44), 41(79)
Odeur : voir plus haut.

Le penténol utilisé comme produit de départ a été obtenu à partir du (+)-(1'R)-2-(2',2',3'-triméthyl-3'-cyclopentén-1'-yl)-2-propén-1-ol par la méthode résumée au Schéma I. Il présentait les caractères analytiques suivants :

Pureté : 98,8%
P. éb. : 95,6°/13 Pa
$[\alpha]^{20}_D$ (pur) = + 77,2°
IR : 3330, 2955, 1640, 1450, 1360, 1060, 890 $cm^{-1}$
RMN($^1H$,360MHz,$CDCl_3$) : 0,74(s, 3H); 1,09(s, 3H) ; 1,59(q, J=2, 3H); 1,65(s large, 1H, OH); 1,74(quint., J=7, 2H); 2,05-2,25(m, 3H); 2,3-2,4(m, 1H); 2,55(t, J=7, 1H); 3,65(t, J=7, 2H); 4,85(s large, 1H); 4,91(d, J=2, 1H); 5,28(s large, 1H) δ ppm
RMN($^{13}C$) : 12,8(q); 21,0(q); 27,0(q); 31,3(t); 32,9(t); 34,0(t); 47,9(s); 56,2(d) ; 62,8(t); 110,7(t); 121,6(d); 147,3(s); 149,5(s) δ ppm
SM : 194($M^+$,35), 179(28), 161(18), 135(99), 119(48), 107(93), 91(100), 79(60), 55(51), 41(92)
Odeur : santalée.

B. A partir de (-)-(S)-1,5,5-triméthyl-4-[1-(vinyloxyméthyl)éthényl)-1-cyclopentène

Le vinyléther susmentionné a été traité thermiquement de façon analogue à celle décrite dans l'Exemple 1B., pour fournir l'aldéhyde désiré, qui présentait des caractères analytiques identiques à ceux décrits sous A.

Le vinyléther de départ a été préparé ainsi.
Un mélange de (-)-(1'S)-2-(2',2',3'-triméthyl-3'-cyclopentén-1'-yl)-2-propén-1-ol (4,15 g, 25 mmole) et $Hg(CH_3COO)_2$ (166 mg, 0,52 mmole) en solution dans du vinyléthyléther (12 g, 166 mmole) a été porté à reflux durant 20 h. L'excès de vinyléthyléther a été distillé ainsi que l'éthanol. Le résidu a été distillé au four à boules en présence de $K_2CO_3$ (332 mg), sous vide poussé, pour donner 2,21 g de (-)-(S)-1,5,5-triméthyl-4-[1-(vinyloxyméthyl)éthényl]-1-cyclopentène (rend. 46%).

P. éb.: 70°/5,3x$10^2$ Pa
$[\alpha]^{20}_D$ (pur) = - 49,7°
IR : 3020, 2990, 2940, 2880, 1640, 1615, 1460, 1330, 1210, 920, 800 $cm^{-1}$
RMN($^1H$,360MHz,$CDCl_3$) : 0,78(s, 3H); 1,08(s, 3H); 1,61(q, J=2, 3H); 2,3(m, 2H); 2,62(t, J=7, 1H); 4,01(dd, J=2, 7, 1H); 4,18(s, 2H); 4,21(dd, J=2, 7, 1H); 5,07(s, 1H); 5,23(q, J=2, 1H); 5,29(s large, 1H); 6,47(dd, J=6, 12, 1H) δ ppm
RMN($^{13}C$) : 12,8(q); 20,9(q); 26,7(q); 33,8(t); 47,7(s); 53,6(d); 71,5(t); 87,1(t); 113,3(t); 121,6(d); 144,9(s); 147,4(s); 151,6(d) δ ppm
SM : 192($M^+$,5), 177(18), 159(14), 148(20), 133(88), 121(25), 119(24), 105(90), 93(100), 91(95), 79(70), 55(32), 41(70)

## Exemple 4

### Préparation de (-)-(1'R)-4-(2',2',3'-triméthyl-3'-cyclopentén-1'-yl)-4-pentén-1-al

Préparé de façon analogue à son énantiomère décrit dans l'Exemple 3 mais à partir de (-)-(1'R)-4-(2',2',3'-triméthyl-3'-cyclopentén-1'-yl)-4-pentén-1-ol ($[\alpha]^{20}_D$ (pur) = - 69,4°) ou de (-)-(1'S)-2-(2',2',3'-triméthyl-3'-cyclopentén-1'-yl)-2-propén-1-ol. Le composé obtenu avait des caractères analytiques identiques à ceux de son énantiomère, à l'exception de :

Pureté : 97,6%
$[\alpha]^{20}_D$ (pur) = - 66,3°
Odeur : voir plus haut.

Exemple 5

Préparation de (-)-(1'R,2S)-2-méthyl-4-(2',2',3'-triméthyl-3'-cyclopentén-1'-yl)-4-pentènenitrile et de (-)-(1'R,2R)-2-méthyl-4-(2',2',3'-triméthyl-3'-cyclopentén-1'-yl)-4-pentènenitrile

Dans un ballon de 500 ml muni d'une agitation magnétique, sous atmosphère d'azote, on a chargé 22,27 g (0,32 mole) d'hydrochlorure d'hydroxylamine, 12,82 g (0,32 mole) de NaOH et 175 ml d'éthanol. On a chauffé à reflux, puis introduit goutte à goutte 60 g (0,29 mole) de (-)-(1'R)-2-méthyl-4-(2',2',3'-triméthyl-3'-cyclopentén-1'-yl)-4-penténal ($[\alpha]^{20}_D$ = -52,8° (pur); voir Exemple 1) et maintenu le reflux pendant 1 h. On a laissé refroidir, repris à l'éther, lavé 1x avec de l'eau, séché et concentré pour obtenir 64,5 g d'oxime de (-)-(1'R)-2-méthyl-4-(2',2',3'-triméthyl-3'-cyclopentén-1'-yl)-4-penténal qui présentait les caractères suivants :

$[\alpha]^{20}_D$(pur) = - 52,2°
IR : 3300, 2959, 2360, 1457, 897 $cm^{-1}$
RMN($^1H$,360MHz) : 0,75(s, 3H); 1,0-1,15(m, 6H); 1,6(s large, 3H) ; 2,0-2,4(m, 4H); 2,45-2,7(m, 2H); 3,30-3,50(m, 1H); 4,88-4,97(m, 2H); 5,27(s large, 1H); 6,49 et 6,53(2d,J=7, 9, 1H) ; 7,31 et 7,35(2d, J=6, 4, 1H) δ ppm
RMN($^{13}C$,360MHz) : 12,81(q); 16,94; 17,43; 17,54; 18,15(4q) ; 21,01(q); 26,90; 26,95(2q); 27,94; 28,14(2d) ; 32,89; 33,06(2d); 33,95; 34,04(2t) ; 41,57; 41,99; 42,26; 42,29(4t); 47,71; 47,75; 47,78; 47,81(4s); 55,35; 55,48; 55,65; 55,86(4d); 112,27; 112,59; 112,89; 113,20(4t); 121,52; 121,63; 121,68(3d); 146,43; 146,63; 147,19; 147,34(4s); 155,98; 156,13; 156,86; 157,06(4d) δ ppm
SM :

isomère 1 : 221(2, $M^+$); 204(25), 188(16), 173(14), 160(16), 148(18), 138(59), 133(40), 119(36), 105(54), 91(80), 79(70), 67(34), 55(45), 41(100), 28(45)
isomère 2 : 221(4, $M^+$) ; 204(39), 188(19), 173(18), 160(21), 148(23), 138(91), 133(52), 119(47), 105(73), 91(100), 79(83), 67(39), 55(50), 41(98), 28(33)
isomère 3 : 204(24), 188(16), 173(13), 160(15), 149(21), 133(36), 119(37), 117(12), 105(61), 91(92), 79(77), 67(33), 65(25), 55(45), 53(37), 51(18), 41(100), 39(64), 28(48)
isomère 4 : 221(1, $M^+$); 204(38), 188(14), 178(4), 173(13), 160(14), 149(18), 133(34), 119(34), 113(14), 105(58), 91(78), 79(72), 67(33), 55(41), 41(100), 28(35)

Odeur : vaguement boisée, santalée

On a chargé dans un ballon 64,5g (0,29 mole) de l'oxime préparée ci-dessus et 60 ml (0,63 mole) d'anhydride acétique et chauffé à reflux pendant 30 min. On a laissé refroidir, repris à l'éther, lavé une fois à l'eau, deux fois avec NaOH à 15% et encore à l'eau jusqu'à neutralité. Après séchage et évaporation, on a obtenu 56,6g de produit brut, dont la purification a fourni le (-)-(1'R)-2-méthyl-4-(2',2',3'-triméthyl-3'-cyclopentén-1'-yl)-4-pentènenitrile (2R/2S 4:6) ayant les caractéristiques suivantes :

Pureté : > 99%
$[\alpha]^{20}_D$ (pur) = -63,4°

Ce produit a été soumis à chromatographie sur colonne de silice, en utilisant comme éluant un mélange cyclohexane/acétate d'éthyle 98:2, pour fournir les deux diastéréomères désirés à l'état pur.

(-)-(1'R,2R)-2-méthyl-4-(2',2',3'-triméthyl-3'-cyclopentén-1'-yl)-4-pentènenitrile

Pureté : 95%
$[\alpha]^{20}_D$ (pur) = -49,0°
IR : 2960, 1640, 1457, 1362, 902 $cm^{-1}$
RMN($^1H$,360MHz) : 0,77(s, 3H) ; 1,09(s, 3H ; 1,32(d, J=7, 3H) ; 1,61(s large, 3H) ; 2,27(m, 3H) ; 2,45(dxd, $J_1$=8, $J_2$=5, 1H) ; 2,53(t, J=9, 1H) ; 2,77(sext, J=7, 1H) ; 5,04(5 large 1H) ; 5,06(s large, 1H) ; 5,27(s large, 1H) δ ppm

RMN($^{13}$C,360Mhz) : 12,9(q) ; 17,9(q) ; 21,0(q) ; 24,5(d) ; 26,8(q) ; 34,0(t) ; 40,8(t) ; 48,1(s) ; 55,9(d) ; 114,3(t) ; 121,3(d) ; 123,0(s) ; 144,9(s) ; 147,4(s) $\delta$ ppm

SM : 203(32,M+) ; 188(78), 173(16), 160(94), 146(22), 133(60), 119(38), 105(92), 91(88), 79(100), 67(28), 55(30), 41(66), 27(24),

(-)-(1'R,2S)-2-méthyl-4-(2',2',3'-triméthyl-3'-cyclopentén-1'-yl)-4-pentènenitrile

Pureté : 95%

$[\alpha]^{20}_D$ (pur) = -65,9°

IR : 2960, 1640, 1457, 1362, 901 $cm^{-1}$

RMN($^1$H,360MHz) : 0,77(s,3H) ; 1,08(s, 3H) ; 1,36(d, J=7, 3H) ; 1,60(s large, 3H) ; 2,24(dxd, $J_1$=5, $J_2$=15, 1H) ; 2,30(m, 2H) ; 2,43(dxd, $J_1$=9, $J_2$=15, 1H) ; 2,52(t, J=9, 1H) ; 2,82(m, 1H) ; 5,05(s large, 1H) ; 5,07(s large, 1H) ; 5,28(s large, 1H) $\delta$ ppm

RMN($^{13}$C,360MHz) : 12,8(q) ; 18,2(q) ; 21,0(q) ; 24,8(d) ; 27,0(q) ; 34,1(t) ; 41,2(t) ; 48,0(s) ; 55,8(d) ; 114,0(t) ; 121,6(d) ; 122,0(s) ; 145,1(s) ; 147,2(s) $\delta$ ppm

SM : 203(32,M+) ; 188(76), 173(17), 160(92), 146(21), 133(60), 119(37), 105(89), 91(86), 79(100), 67(28), 55(30), 41(63), 27(20)

Exemple 6

Préparation de (-)-(1'R)-2-méthyl-4-(2',2',3'-triméthyl-3'-cyclopentén-1'-yl)-4-pentén-1-ol

On a ajouté à l'aldéhyde obtenu selon l'Exemple 1 (2R/2S 60 : 40) de l'éthanol (40 ml) et ensuite $NaBH_4$ (235 mg, 6 mmole). Après 45 min, on a hydrolysé en versant sur glace, extrait à l'éther de pétrole 30/50, lavé à l'eau jusqu'à neutralité, séché, concentré et distillé pour obtenir le penténol désiré sous forme d'un mélange de 2 diastéréomères 2R/2S 55 : 45 (rend. 77%).

Le produit ainsi obtenu avait des caractères analytiques similaires à ceux décrits dans l'art antérieur, à l'exception de :

$[\alpha]^{20}_D$ (pur) = - 69,7°

Les deux diastéréomères présents dans ce mélange ont été séparés par "flash" chromatographie sur 230 g de $SiO_2$ avec, comme éluant, un mélange de cyclohexane/acétate d'éthyle 95 : 5. On a ainsi obtenu :

(-)-(1'R,2S)-2-méthyl-4-(2',2',3'-triméthyl-3'-cyclopentén-1'-yl)-4-pentén-1-ol

(pureté 93%)

$[\alpha]^{20}_D$ = - 55,7°; c=1,2, $CHCl_3$

(-)-(1'R,2R)-2-méthyl-4-(2',2',3'-triméthyl-3'-cyclopentén-1'-yl)-4-pentén-1-ol

(pureté 70%)

$[\alpha]^{20}_D$ = - 75,4°; c=1,4, $CHCl_3$

Une séparation chromatographique du même mélange sur colonne de type Carbowax ® 20 M, 15%, 15 m, 0,25 mm de diamètre, à 160° a permis d'obtenir lesdits diastéréomères avec les caractéristiques suivantes :

(-)-(1'R,2S)

$R_t$ =7,2 min

$[\alpha]^{20}_D$ = - 60,8°; c=0,6, $CCl_4$

RMN($^1H$,360MHz,$CDCl_3$) : 0,76(s, 3H); 0,96(d, J=7, 3H); 1,10(s, 3H); 1,5(s large, 1H, OH); 1,61(s large, 3H); 1,9-2,04(m, 2H); 2,12-2,23(m, 2H); 2,30-2,39(m, 1H); 2,59(t, J=7, 1H); 3,43(dd, J=10, 6, 1H); 3,54(dd, J=10, 6, 1H); 4,92(s, 1H); 4,95(s, 1H); 5,28(s, 1H) δ ppm

RMN($^{13}C$) : 12,8(q); 17,3(q); 21,0(q); 27,0(q); 34,2(t); 34,2(d); 41,9(t); 47,8(s); 55,7(d); 68,1(t); 112,0(t); 121,6(d); 147,4(s); 148,6(s) δ ppm

(-)-(1'R,2R)

$R_t$ = 7,6 min
$[\alpha]^{20}_D$ = - 73,1°; c=0,5, $CCl_4$
RMN($^1H$,360MHz,$CDCl_3$) : 0,76(s, 3H); 0,89(d, J=7, 3H); 1,10(s, 3H).; 1,58(s large, 1H); 1,61(d, J=2, 3H); 1,8-1,97(m, 2H); 2,12-2,40(m, 3H); 2,54(t, J=8, 1H); 3,48(dd, J=10, 5, 1H); 3,54(dd, J=10, 5, 1H); 4,92(s, 2H); 5,28(s, 1H) δ ppm
RMN($^{13}C$) : 12,8(q); 16,3(q); 21,0(q); 27,0(q); 34,2(d); 34,3(t);41,4(t); 47,8(s); 55,2(d); 68,6(t); 112,1(t); 121,6(d); 147,4(s); 148,0(s) δ ppm

Exemple 7

Préparation de (+)-(1'S)-2-méthyl-4-(2',2',3'-triméthyl-3'-cyclopentén-1'-yl)-4 - pentén-1-ol

Obtenu à partir de l'aldéhyde décrit dans l'Exemple 2, en suivant le mode opératoire décrit à l'Exemple 6. On a obtenu le penténol désiré sous forme d'un mélange de diastéréomères 2R/2S 55 : 45 et $[\alpha]^{20}_D$ (pur) = + 79,1°. Les deux diastéréomères ont été séparés par chromatographie en phase gazeuse sur colonne de type Carbowax ® 20 M, 15%, 6m, 6 mm de diamètre, à 180°.

(+)-(1'S,2S)-2-méthyl-4-(2',2',3'-triméthyl-3'-cyclopentén-1'-yl)-4-pentén-1-ol

$[\alpha]^{20}_D$ = + 67,6°; c=0,15, $CCl_4$

(+)-(1'S,2R)-2-méthyl-4-(2',2',3'-triméthyl-3'-cyclopentén-1'-yl)-4-pentén-1-ol

$[\alpha]^{20}_D$ = + 71,7°; c=1,0, $CCl_4$

Exemple 8

Préparation d'une composition parfumante

On a préparé une composition parfumante de base en mélangeant les ingrédients suivants :

| Ingrédients | Parties en poids |
|---|---|
| Acétate de benzyle | 20 |
| Acétate de géranyle | 5 |
| Acétate de styrallyle | 5 |
| Aldéhyde hexylcinnamique | 20 |
| Glycolate d'allyle amyle à 10%* | 30 |
| Essence de bergamote | 90 |
| Essence de citron | 10 |
| Citronellol | 10 |
| Coumarine | 35 |
| Dihydromyrcénol [1)] | 5 |
| Galaxolide ® [2)] 50 | 20 |
| Géraniol | 5 |
| Hedione ® [3)] | 140 |
| Indol à 10%* | 5 |
| Isoeugénol | 5 |
| Absolu de jasmin | 20 |
| α-Isométhylionone | 110 |
| Muscone | 25 |
| Nérol | 5 |
| p-Crésol à 1%* | 20 |
| Phénéthylol | 10 |
| Essence de baies de piment | 10 |
| Salicylate de benzyle | 15 |
| Salicylate d'hexyle | 10 |
| Salicylate de pipol | 10 |
| Essence de santal | 190 |
| Undécalactone | 5 |
| Vanilline à 10%* | 25 |
| Essence d'Ylang | 40 |
| Total | 900 |

* dans le dipropylèneglycol
1) 2,6-dimèthyl-7-octèn-2-ol; origine: International Flavors & Fragrances, USA
2) 1,3,6,7-hexahydro-4,6,6,7,8,8-hexamèthyl-cyclopenta[g]isochromène; origine: International Flavors & Fragrances, USA
3) dihydrojasmonate de mèthyle; origine: Firmenich SA, Genève, Suisse

A cette composition de base, de type boisé-oriental, on a ajouté 100 parties en poids de (-)-(1'R)-2-méthyl-4-(2',2',3'-triméthyl-3'-cyclopentén-1'-yl)-4-pentén-1-al. La composition nouvelle ainsi obtenue développait une odeur dont la note santalée se trouvait nettement exaltée par rapport à la composition de base et avait par ailleurs acquis un caractère très frais et puissant en tête. En plus, on décelait maintenant une connotation ozonée, marine et aldéhydée, totalement absente de l'odeur de la composition de base.
L'addition à la composition de base de la même quantité de (+)-(1'S)-2-méthyl-4-(2',2',3'-triméthyl-3'-cyclopentén-1'-yl)-4-pentén-1-al provoque également un effet d'exaltation de la note santalée, la composition nouvelle ayant par ailleurs acquis un caractère aldéhydé-métallique, mais l'effet marin n'était plus observé.
Lorsqu'on a ajouté à la composition de base 100 parties en poids de (+)-(1'R)-4-(2',2',3'-triméthyl-3'-cyclopentén-1'-yl)-4-pentén-1-al, on n'a plus observé un renforcement de la note santalée, mais la composition avait alors acquis une note nettement plus fleurie-verte, l'arrière-note hespéridée étant aussi beaucoup plus puissante.
L'addition à la composition de base de la même quantité de (-)-(1'S)-4-(2',2',3'-triméthyl-3'-cyclopentén-1'-yl)-4-pentén-1-al a alors nettement affaibli le caractère santalé de la composition, laquelle avait acquis une note beaucoup plus aldéhydée et presque savonneuse.

Exemple 9

Préparation d'une composition parfumante

On a préparé une composition parfumante de base en mélangeant les ingrédients suivants :

| Ingrédients | Parties en poids |
|---|---|
| Acétate de benzyle | 100 |
| Acétate de pipol | 10 |
| Aldéhyde hexylcinnamique | 110 |
| Alcool cinnamique | 200 |
| Essence de galbanum à 10%* | 30 |
| Hedione ® 1) | 40 |
| Indol à 10%* | 25 |
| Jasmonate de méthyle | 10 |
| Méthylisoeugénol | 65 |
| Mayol® 2) | 25 |
| 1-(5,5-Diméthyl-1-cyclohexén-1-yl)-4-pentén-1-one 3) | 55 |
| Phénéthylol | 215 |
| (Z)-3-Hexén-1-ol à 10%* | 20 |
| α-Terpinéol | 20 |
| Zestover ® 4) à 10%* | 25 |
| Total | 950 |

* dans le dipropylèneglycol
1) voir exemple prècèdent
2) cis-4-(1-mèthylèthyl)-cyclohexanol; origine: Firmenich SA, Genève, Suisse
3) Neobutenone ®; origine: Firmenich SA, Genève, Suisse
4) 2,4-dimèthyl-3-cyclohexène-1-carbaldèhyde; origine: Firmenich SA, Genève, Suisse

A cette composition de base, de type floral-jacinthe, on a ajouté 50 parties en poids de (-)-(1'R)-2-méthyl-4-(2',2',3'-triméthyl-3'-cyclopentén-1'-yl)-4-pentén-1-al pour obtenir une nouvelle composition dont la note verte était

beaucoup plus aqueuse et fraîche que celle de la composition de base et aussi accompagnée d'une sous-note boisée très naturelle. Cet effet frais, marin ne pouvait plus être perçu lorsqu'on a ajouté à la composition de base la même quantité de (+)-(1'S)-2-méthyl-4-(2',2',3'-triméthyl-3'-cyclopentén-1'-yl)-4-pentén-1-al.
L'addition à la composition de base de 50 parties en poids de (-)-(1'R)-4-(2',2',3'-triméthyl-3'-cyclopentén-1'-yl)-4-pentén-1-al lui impartit un caractère plus épicé et ajoute une légère note métallique, un peu citronnelle.
Pour sa part, le (+)-(1'S)-4-(2',2',3'-triméthyl-3'-cyclopentén-1'-yl)-4-pentén-1-al, lorsqu'ajouté à la composition dans la même quantité, valorise la qualité olfactive de cette dernière, en renforçant les notes vertes, lesquelles sont alors accompagnées d'un léger côté métallique.

D'autre part, lorsqu'on a ajouté à la composition de base 50 parties en poids de (-)-(1'R,2R)-2-méthyl-4-(2',2',3'-triméthyl-3'-cyclopentén-1'-yl)-4-pentènenitrile, on a obtenu une composition nouvelle dont le caractère olfactif était tout à fait distinct de celui des compositions ci-dessus, son odeur étant beaucoup plus terreuse, moins verte et moins boisée, avec une légère sous-note hespéridée. L'addition de ce composé a pour effet d'estomper l'aspect floral de la composition de base, tout en lui impartissant un caractère nettement plus prothe de l'odeur de l'absolu de jacinthe.

Exemple 10

Préparation d'une composition parfumante

On a préparé une composition parfumante de base en mélangeant les ingrédients suivants :

| Ingrédients | Parties en poids |
|---|---|
| Aldéhyde décyclique à 10%* | 10 |
| Aldéhyde undécylénique à 10%* | 40 |
| Aldéhyde dodécyclique à 10%* | 10 |
| Aldéhyde méthylnonylacétique à 10%* | 10 |
| Essence de racines d'Angélique à 10%* | 10 |
| Castoréum à 10%* | 20 |
| Civette naturelle dégraissée à 10%* | 10 |
| Galbanum résinoïde | 10 |
| Absolu de jasmin à 50%* | 100 |
| Essence d'oliban | 10 |
| Patchouli | 30 |
| Acétate de styrallyle | 15 |
| $\alpha$-Isométhylionone | 95 |
| Essence de coriandre | 5 |
| Hydroxycitronellol | 65 |
| Cyclopentadécanolide à 10%* | 50 |
| Jasmin synthétique | 100 |
| Bergamote synthétique | 100 |
| Citron synthétique | 40 |
| Mousse de chêne absolu à 50%* | 30 |
| Néroli synthétique | 20 |
| Muscone à 10%* | 50 |
| Coumarine | 50 |
| Musc ambrette | 10 |
| Musc cétone | 30 |
| Phtalate de diéthyle | 50 |
| Total | 970 |

* dans le phtalate de diéthyle

A 98 g de cette composition de base de type boisé-animal, on a ajouté 2 g de chacun des isomères chiraux du 2-méthyl-4-(2',2',3'-triméthyl-3'-cydopentén-1'-yl)-4-pentén-1-ol indiqués au Tableau ci-après, pour préparer les quatre compositions parfumantes B à E.

TABLEAU

| Composition | Isomère |
|---|---|
| A | (-)-(1'R) |
| B | (-)-(1'R,2R) |

TABLEAU (suite)

| Composition | Isomère |
|---|---|
| C | (-)-(1'R,2S) |
| D | (+)-(1'S,2S) |
| E | (+)-(1'S,2R) |

Les quatre compositions B à E ont ensuite été évaluées à l'aveugle par un panel d'experts parfumeurs, lesquels devaient se prononcer sur la qualité de l'odeur, sa puissance et son analogie avec l'essence de santal naturelle. Les parfumeurs ont unanimement choisi la composition B comme étant celle dont l'odeur santalée était la plus puissante et naturelle, avec un caractère lait de santal très net, et une note odorante très élégante. Pour ce qui était des trois autres compositions, les parfumeurs ont placé la composition C en deuxième position, tout en jugeant que son odeur santalée avait un caractère beaucoup moins naturel, moins lait de santal, alors qu'elle possédait un caractère cèdre qui n'était pas présent dans la composition B.
D'autre part, les avis étaient partagés vis-à-vis des compositions D et E, le seul consensus étant qu'elles ne semblaient guère se différencier de la composition de base du point de vue olfactif.
Lorsque le même panel a évalué à l'aveugle la composition B par rapport à la composition A, on a observé de nouveau une préférence nette pour la composition B, son odeur ayant été jugée encore plus puissante et naturelle, plus élégante dans son caractère santalé.

**Revendications**

**1.** Composé de formule

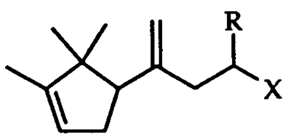

(I)

dans laquelle R représente l'hydrogène ou un radical méthyle et X représente un groupe - CHO ou - C ≡ N.

**2.** Composé selon la revendication 1, sous forme d'un isomère optiquement actif sélectionné dans le groupe constitué par :

a. (-)-(1'R)-2-methyl-4-(2',2',3'-trimethyl-3'-cyclopenten-1'-yl)-4-penten-1-al;
b. (+)-(1'S)-2-méthyl-4-(2',2',3'-triméthyl-3'-cyclopentén-1'-yl)-4-pentén-1-al;
c. (-)-(1'R)-4-(2',2',3'-trimethyl-3'-cyclopenten-1'-yl)-4-penten-1-al;
d. (+)-(1'S)-4-(2',2',3'-triméthyl-3'-cyclopentén-1'-yl)-4-pentén-1-al;
e. (-)-(1'R,2S)-2-méthyl-4-(2',2',3'-triméthyl-3'-cyclopentén-1'-yl)-4-pentènenitrile; et
f. (-)-(1'R,2R)-2-méthyl-4-(2',2',3'-triméthyl-3'-cyclopentén-1'-yl)-4-pentènenitrile;

ou sous forme de tout mélange de deux ou plusieurs parmi les composés cités ci-dessus.

**3.** Procédé pour la préparation d'un composé de formule

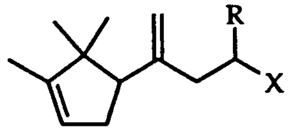

(I)

dans laquelle R représente l'hydrogène ou un radical méthyle et X représente un groupe - CHO ou - C ≡ N, caractérisé en ce qu'on soumet un éther de formule

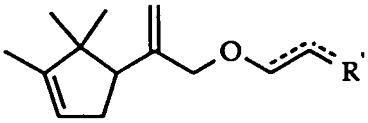

(II)

possédant une double liaison dans l'une des positions indiquées par les lignes pointillées et dans laquelle R' représente l'hydrogène, un radical méthyle ou un radical méthylène, à un traitement thermique pour former un aldéhyde de formule

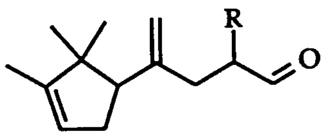

(I')

dans laquelle R représente l'hydrogène ou un radical méthyle et, le cas échéant, on transforme ledit aldéhyde en le nitrile correspondant, de façon connue en soi.

4. Procédé selon la revendication 3, caractérisé en ce que l'éther de formule (II) est présent sous forme de l'un de ses énantiomères de configuration (R) ou (S), et en ce qu'on obtient l'énantiomère correspondant du composé (I).

5. Procédé selon la revendication 4, caractérisé en ce qu'on soumet le 4-[1-(allyloxyméthyl)éthényl]-1,5,5-triméthyl-1-cyclopentène à un traitement thermique, en présence d'un catalyseur, pour obtenir le 2-méthyl-4-(2,2,3-triméthyl-3-clopentén-1-yl)-4-pentén-1-al.

6. Procédé selon la revendication 5, caractérisé en ce qu'on utilise le 4-[1-(allyloxyméthyl)éthényl]-1,5,5-triméthyl-1-cyclopentène sous forme de l'un de ses énantiomères de configuration (-)-(S) ou (+)-(R) pour obtenir le (-)-(1'R)-, ou respectivement (+)-(1'S)-2-méthyl-4-(2',2',3'-triméthyl-3'-cyclopentén-1'-yl)-4-pentén-1-al.

7. Procédé selon la revendication 5 ou 6, caractérisé en ce que le catalyseur est le dichlorure de tris(triphénylphosphine)ruthénium.

8. Procédé selon la revendication 5 ou 6, caractérisé en ce que le catalyseur est constitué par du ruthénium sur charbon et la réaction est effectuée dans le mésitylène.

9. Utilisation d'un composé selon la revendication 1 ou 2, à titre d'ingrédient parfumant.

10. Composition parfumante ou article parfumé contenant à titre d'ingrédient actif au moins un composé selon la revendication 1 ou 2.

11. A titre d'article parfumé selon la revendication 10, un parfum ou une eau de toilette, un savon, un gel de douche ou bain, une préparation cosmétique, un shampoing ou autre produit d'hygiène capillaire, un désodorisant corporel ou d'air ambiant, un détergent ou un adoucissant textile, ou un produit d'entretien.

12. Procédé pour conférer, modifier ou renforcer le caractère odorant de type marin, ozoné, aqueux d'une composition parfumante ou d'un article parfumé, caractérisé en ce qu'on ajoute à ladite composition ou audit article un composé selon la revendication 2a. ou 2d. en quantité suffisante pour obtenir l'effet odorant de type susmentionné.

13. Utilisation d'un composé de formule

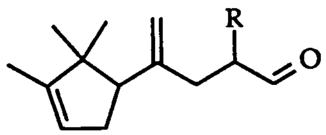

(I')

dans laquelle R représente l'hydrogène ou un radical méthyle, à titre de produit de départ pour la préparation du nitrile correspondant, caractérisée en ce qu'on transforme, de façon connue en soi, ledit produit de départ en l'oxime correspondante et en ce qu'on fait réagir cette dernière avec un agent déshydratant.

**14.** Utilisation selon la revendication 13, caractérisée en ce que ledit composé de formule (I') se présente sous forme de l'un de ses isomères chiraux de configuration (1'R).

**15.** Oxime de 2-méthyl-4-(2,2,3-triméthyl-3-cyclopentén-1-yl)-4-penténal.

**16.** Utilisation d'un composé de formule

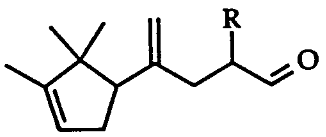

(I')

dans laquelle R représente l'hydrogène ou un radical méthyle, à titre de produit de départ pour la préparation de l'alcool correspondant, caractérisée en ce qu'on traite ledit composé avec un agent réducteur.

**17.** Utilisation selon la revendication 16, du 2-méthyl-4-(2,2,3-triméthyl-3-cyclopentén-1-yl)-4-pentén-1-al pour la préparation du 2-méthyl-4-(2,2,3-triméthyl-3-cyclopentén-1-yl)-4-pentén-1-ol.

**18.** Utilisation selon la revendication 17, caractérisée en ce qu'on emploie le 2-méthyl-4-(2,2,3-triméthyl-3-cyclopentén-1-yl)-4-pentén-1-al sous forme de l'un de ses isomères optiquement actifs de configuration (-)-(1'R) ou (+)-(1'S) pour obtenir le (-)-(1'R)- ou respectivement (+)-(1'S)-2-méthyl-4-(2',2',3'-triméthyl-3'-cyclopentén-1'-yl)-4-pentén-1-ol.

**19.** Ether de formule

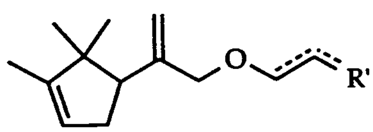

(II)

ayant une double liaison dans l'une des positions indiquées par les lignes pointillées et dans laquelle R' représente l'hydrogène, un radical méthyle ou un radical méthylène, ou l'un de ses énantiomères de configuration (R) ou (S).

**20.** 4-[1-(Allyloxyméthyl)éthényl]-1,5,5-triméthyl-1-cyclopentène ou l'un de ses énantiomères de configuration (-)-(S) ou (+)-(R).

**21.** Composé choisi dans le groupe constitué par

a. (+)-(1'S,2R)-2-méthyl-4-(2',2',3'-triméthyl-3'-cyclopentén-1'-yl)-4-pentén-1-ol;
b. (+)-(1'S,2S)-2-méthyl-4-(2',2',3'-triméthyl-3'-cyclopentén-1'-yl)-4-pentén-1-ol;
c. (-)-(1'R,2R)-2-méthyl-4-(2',2',3'-triméthyl-3'-cyclopentén-1'-yl)-4-pentén-1-ol;
d. (-)-(1'R,2S)-2-méthyl-4-(2',2',3'-triméthyl-3'-cyclopentén-1'yl)-4-pentén-1-ol.

**22.** Utilisation d'un composé selon la revendication 21, à titre d'ingrédient parfumant.

## Claims

**1.** A compound of formula

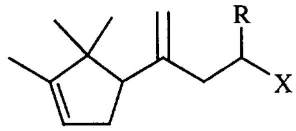

(I)

wherein R represents a hydrogen atom or a methyl radical and X stands for a - CHO, or - C ≡ N group.

**2.** The compound of claim 1, in the form of an optically active isomer selected from the group consisting of

a. (-)-(1'R)-2-methyl-4-(2',2',3'-trimethyl-3'-cyclopenten-1'-yl)-4-penten-1-al ;
b. (+)-(1'S)-2-methyl-4-(2',2',3'-trimethyl-3'-cyclopenten-1'-yl)-4-penten-1-al ;
c. (-)-(1'R)-4-(2',2',3'-trimethyl-3'-cyclopenten-1'-yl)-4-penten-1-al ;
d. (+)-(1'S)-4-(2',2',3'-trimethyl-3'-cyclopenten-1'-yl)-4-penten-1-al ;
e. (-)-(1'R,2S)-2-methyl-4-(2',2',3'-trimethyl-3'-cyclopenten-1'-yl)-4-pentenenitrile ; and
f. (-)-(1'R,2R)-2-methyl-4-(2',2',3'-trimethyl-3'-cyclopenten-1'-yl)-4-pentenenitrile ;

or in the form of a mixture of any two or more compounds selected in the group defined in claim 2.

**3.** A process for the preparation of a compound of formula

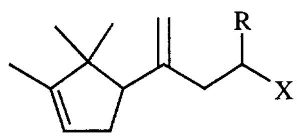

(I)

wherein R represents hydrogen or a methyl radical and X stands for a - CHO, or - C ≡ N group, which process comprises thermally treating an ether of formula

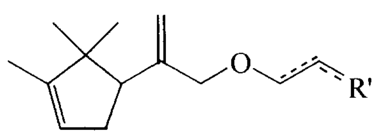

(II)

having a double bond in one of the positions indicated by the dotted lines and wherein R' represents hydrogen, a methyl radical or a methylene radical, to form an aldehyde of formula

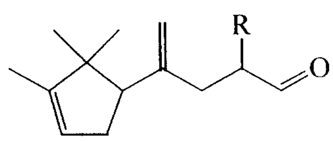

(I')

wherein R represents hydrogen or a methyl radical, and, if necessary, converting said aldehyde into the corresponding nitrile, in a generally known manner.

**4.** The process of claim 3, wherein there is used an ether of formula (II) in the form of one of its enantiomers of (R) or

(S) configuration, so as to obtain the corresponding enantiomer of compound (I).

5. The process of claim 4, wherein 4-[1-(allyloxymethyl)ethenyl]-1,5,5-trimethyl-1-cyclopentene is thermally treated in the presence of a catalyst, to provide 2-methyl-4-(2,2,3-trimethyl-3-cyclopenten-1-yl)-4-penten-1-al.

6. The process of claim 5, wherein 4-[1-(allyloxymethyl)ethenyl]-1,5,5-trimethyl-1-cyclopentene is used in the form of its enantiomer of (-)-(S) or (+)-(R) configuration, to provide (-)-(1'R)-, or respectively (+)-(1'S)-2-methyl-4-(2',2',3-trimethyl-3'-cyclopenten-1'-yl)-4-penten-1-al.

7. The process of claim 5 or 6, wherein the catalyst is tris(triphenylphosphine)ruthenium dichloride.

8. The process of claim 5 or 6, wherein the catalyst is ruthenium on carbon and the reaction is carried out in mesitylene.

9. Use as a perfuming ingredient of a compound according to claim 1 or 2.

10. A perfuming composition or a perfumed article, containing as an active ingredient at least one compound according to claim 1 or 2.

11. A perfumed article according to claim 10, in the form of a perfume or a cologne, a soap, a bath or shower gel, a cosmetic preparation, a shampoo or other hair-care product, a body deodorant or an air-freshener, a detergent, a fabric softener or a household product.

12. A method to confer, improve, enhance or modify the marine, ozone, watery type odor character of a perfuming composition or a perfumed article, which method comprises adding thereto a compound according to claim 2a. or 2d., in an amount sufficient to achieve the fragrant effect of the type above-mentioned.

13. Use of a compound of formula

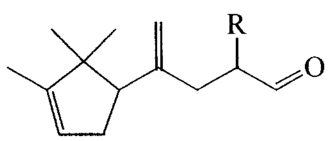

(I')

wherein R represents hydrogen or a methyl radical, as starting product for the preparation of the corresponding nitrile, characterized in that the said starting product is converted, in a generally known manner, into the corresponding oxime and the latter is then reacted with a dehydrating agent to form the corresponding nitrile.

14. Use according to claim 13, wherein said aldehyde of formula (I') is in the form of a chiral isomer of (1'R) configuration.

15. 2-Methyl-4-(2,2,3-trimethyl-3-cyclopenten-1-yl)-4-pentenal oxime.

16. Use of a compound of formula

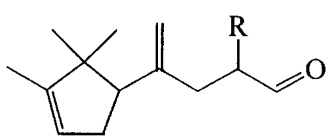

(I')

in which R represents hydrogen or a methyl radical, as starting product for the preparation of the corresponding alcohol, characterized in that the said compound is treated with a reducing agent.

17. Use according to claim 16, characterized in that 2-methyl-4-(2,2,3-trimethyl-3-cyclopenten-1yl)-4-penten-1-al is

converted into 2-methyl-4-(2,2,3-trimethyl-3-cyclopenten-1-yl)-4-penten-1-ol.

**18.** Use according to claim 17, characterized in that 2-methyl-4-(2,2,3-trimethyl-3-cyclopenten-1 -yl)-4-penten-1-al is used in form of one of its optically active isomers of configuration (-)-(1'R) or (+)-(1'S), to obtain (-)-(1'R) or respectively, (+)-(1'S)-2-methyl-4-(2',2',3'-trimethyl-3-cyclopenten-1-yl)-4-penten-1-ol.

**19.** An ether of formula

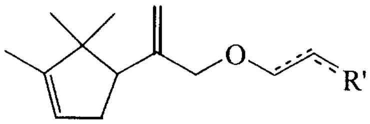

(II)

having a double bond in one of the positions indicated by the dotted lines and wherein R' represents hydrogen, a methyl radical or a methylene radical, or one of its enantiomers of (R) or (S) configuration.

**20.** 4-[1-(Allyloxymethyl)ethenyl]-1,5,5-trimethyl-1-cyclopentene or one of its enantiomers of (-)-(S) or (+)-(R) configuration.

**21.** A compound selected from the group consisting of

a. (+)-(1'S,2R)-2-methyl-4-(2',2',3'-trimethyl-3'-cyclopenten-1'-yl)-4-penten-1-ol ;
b. (+)-(1'S,2S)-2-methyl-4-(2',2',3'-trimethyl-3'-cyclopenten-1'-yl)-4-penten-1-ol ;
c. (-)-(1'R,$^2$R)-2-methyl-4-(2',2',3'-trimethyl-3'-cyclopenten-1'-yl)-4-penten-1-ol ; and
d. (-)-(1'R,1S)-2-methyl-4-(2',2',3'-trimethyl-3'-cyclopenten-1'-yl)-4-penten-1-ol.

**22.** Use as a perfuming ingredient of a compound according to claim 21.

**Patentansprüche**

**1.** Verbindung mit der Formel

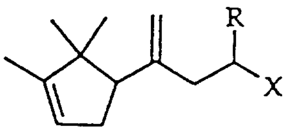

(I),

in der R für Wasserstoff oder einen Methylrest und X für eine Gruppe -CHO oder -C≡N steht.

**2.** Verbindung nach Anspruch 1 in Form eines optisch aktiven Isomers, das aus der Gruppe ausgewählt ist bestehend aus:

a. (-)-(1'R)-2-Methyl-4-(2',2',3'-trimethyl-3'-cyclopenten-1'-yl)-4-penten-1-al;
b. (+)-(1'S)-2-Methyl-4-(2',2',3'-trimethyl-3'-cyclopenten-1'-yl)-4-penten-1-al;
c. (-)-(1'R)-4-(2',2',3'-Trimethyl-3'-cyclopenten-1'-yl)-4-penten-1-al;
d. (+)-(1'S)-4-(2',2',3'-Trimethyl-3'-cyclopenten-1'-yl)-4-penten-1-al;
e. (-)-(1'R,2S)-2-Methyl-4-(2',2',3'-trimethyl-3'-cyclopenten-1'-yl)-4-pentennitril; und
f. (-)-(1'R,2R)-2-Methyl-4-(2',2',3'-trimethyl-3'-cyclopenten-1'-yl)-4-pentennitril;

oder in Form von jeglicher Mischung von zwei oder mehr der oben genannten Verbindungen.

**3.** Verfahren für die Herstellung einer Verbindung mit der Formel

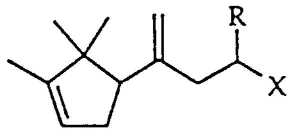

(I),

in der R für Wasserstoff oder einen Methylrest und X für eine Gruppe -CHO oder -C≡N steht, dadurch gekennzeichnet, daß ein Ether mit der Formel

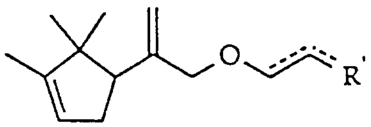

(II),

der eine Doppelbindung in einer der mit den gepunkteten Linien bezeichneten Positionen aufweist, und in der R' für Wasserstoff, einen Methylrest oder einen Methylenrest steht, einer Wärmebehandlung unterzogen wird, um einen Aldehyd mit der Formel

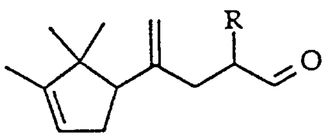

(I')

zu bilden, in der R für Wasserstoff oder einen Methylrest steht und der Aldehyd gegebenenfalls auf an sich bekannte Weise in das entsprechende Nitril überführt wird.

4. Verfahren nach Anspruch 3, dadurch gekennzeichnet, daß der Ether mit der Formel (II) in Form eines seiner Enantiomere mit der (R)- oder (S)-Konfiguration vorliegt, sowie dadurch, daß das entsprechende Enantiomer der Verbindung (I) hergestellt wird.

5. Verfahren nach Anspruch 4, dadurch gekennzeichnet, daß 4-[1-(Allyloxymethyl)ethenyl]-1,5,5-trimethyl-1-cyclopenten einer Wärmebehandlung in Gegenwart eines Katalysators unterzogen wird, um 2-Methyl-4-(2,2,3-trimethyl-3-cyclopenten-1-yl)-4-penten-1-al herzustellen.

6. Verfahren nach Anspruch 5, dadurch gekennzeichnet, daß 4-[1-(Allyloxymethyl)ethenyl]-1,5,5-trimethyl-1-cyclopenten in Form eines seiner Enantiomere mit der Konfiguration (-)-(S) oder (+)-(R) für die Herstellung von (-)-(1'R)- bzw. (+)-(1'S)-2-Methyl-4-(2',2',3'-trimethyl-3'-cyclopenten-1'-yl)-4-penten-1-al eingesetzt wird.

7. Verfahren nach Anspruch 5 oder 6, dadurch gekennzeichnet, daß der Katalysator Tris(triphenylphosphin)rutheniumdichlorid ist.

8. Verfahren nach Anspruch 5 oder 6, dadurch gekennzeichnet, daß der Katalysator aus Ruthenium auf Kohle besteht und die Umsetzung in Mesitylen durchgeführt wird.

9. Verwendung einer Verbindung nach Anspruch 1 oder 2 als Riechstoff.

10. Riechstoffzusammensetzung oder parfümierter Artikel mit mindestens einer Verbindung nach Anspruch 1 oder 2 als wirksamem Inhaltsstoff.

11. Als parfümierter Artikel nach Anspruch 10, ein Parfüm oder ein Eau de Toilette, eine Seife, ein Dusch- oder Badegel, eine kosmetische Zubereitung, ein Shampoo oder ein weiteres Haarpflegeprodukt, ein Körper- oder Raumluftdeodorant, ein Detergens oder ein Weichspüler für Textilien, oder ein Universalreiniger.

**12.** Verfahren zum Verleihen, Modifizieren oder Verstärken des meer-, ozon- oder wasserartigen Geruchscharakters einer Riechstoffzusammensetzung oder eines parfümierten Artikels, dadurch gekennzeichnet, daß der Zusammensetzung bzw. dem Artikel eine Verbindung nach Anspruch 2a. oder 2d. in einer ausreichenden Menge zugegeben wird, um den Geruchseffekt von dem oben genannten Typ zu erhalten.

**13.** Verwendung einer Verbindung mit der Formel

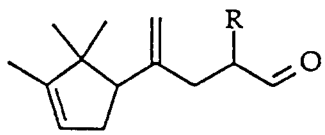

(I'),

in der R für Wasserstoff oder einen Methylrest steht, als Ausgangsprodukt für die Herstellung des entsprechenden Nitrils, dadurch gekennzeichnet, daß das Ausgangsprodukt auf an sich bekannte Weise in das entsprechende Oxim überführt wird, und daß dieses letztere mit einem Dehydratisierungsmittel umgesetzt wird.

**14.** Verwendung nach Anspruch 13, dadurch gekennzeichnet, daß die Verbindung mit der Formel (I') in Form eines seiner chiralen Isomere mit der (1'R)-Konfiguration vorliegt.

**15.** 2-Methyl-4-(2,2,3-trimethyl-3-cyclopenten-1-yl)-4-pentenaloxim.

**16.** Verwendung einer Verbindung mit der Formel

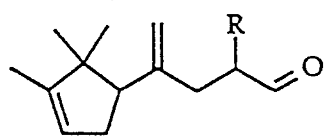

(I'),

in der R für Wasserstoff oder einen Methylrest steht, als Ausgangsprodukt für die Herstellung des entsprechenden Alkohols, dadurch gekennzeichnet, daß die Verbindung mit einem Reduktionsmittel behandelt wird.

**17.** Verwendung nach Anspruch 16 von 2-Methyl-4-(2,2,3-trimethyl-3-cyclopenten-1-yl)-4-penten-1-al für die Herstellung von 2-Methyl-4-(2,2,3-trimethyl-3-cyclopenten-1-yl)-4-penten-1-ol.

**18.** Verwendung nach Anspruch 17, dadurch gekennzeichnet, daß 2-Methyl-4-(2,2,3-trimethyl-3-cyclopenten-1-yl)-4-penten-1-al in Form eines seiner optisch aktiven Isomere mit der Konfiguration (-)-(1'R) oder (+)-(1'S) eingesetzt wird, um (-)-(1'R)- bzw. (+)-(1'S)-2-Methyl-4-(2',2',3'-trimethyl-3'-cyclopenten-1'-yl)-4-penten-1-ol herzustellen.

**19.** Ether mit der Formel

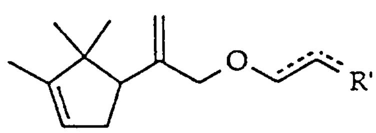

(II),

mit einer Doppelbindung in einer der durch die gepunkteten Linien angedeuteten Positionen, und in der R' für Wasserstoff, einen Methylrest oder einen Methylenrest steht, oder eines seiner Enantiomere mit der (R)- oder (S)-Konfiguration.

**20.** 4-[1-(Allyloxymethyl)ethenyl]-1,5,5-trimethyl-1-cyclopenten oder eines seiner Enantiomere mit der Konfiguration (-)-(S) oder (+)-(R).

**21.** Verbindung, ausgewählt aus der Gruppe bestehend aus

a. (+)-(1'S,2R)-2-Methyl-4-(2',2',3'-trimethyl-3'-cyclopenten-1'-yl)-4-penten-1-ol;
b. (+)-(1'S,2S)-2-Methyl-4-(2',2',3'-trimethyl-3'-cyclopenten-1'-yl)-4-penten-1-ol;
c. (-)-(1'R,2R)-2-Methyl-4-(2',2',3'-trimethyl-3'-cyclopenten-1'-yl)-4-penten-1-ol;
d. (-)-(1'R,2S)-2-Methyl-4-(2',2',3'-trimethyl-3'-cyclopenten-1'-yl)-4-penten-1-ol.

**22.** Verwendung einer Verbindung nach Anspruch 21 als Riechstoff.